# EUROPÄISCHE PATENTANMELDUNG

(11) **EP 0 911 047 A1**
(43) Veröffentlichungstag der Anmeldung: **28.04.1999**
(21) Anmeldenummer: 98123206.9
(22) Anmeldetag: 28.05.1994
(51) Int. Cl.: A61M 15/00, B65D 83/04

(54) **Kapselhalterung**

(30) Priorität: 03.06.1993 DE 4318455
(62) Teilanmeldung aus: 95901982.9
(71) Anmelder: Boehringer Ingelheim Pharma KG, 55216 Ingelheim am Rhein (DE)
(72) Erfinder: Die Erfindernennung liegt noch nicht vor

(57) **Zusammenfassung**

Die Erfindung betrifft einen Inhalator für die Inhalation pulverförmiger Arzneimittel aus Kapseln.

## Beschreibung

Die Erfindung betrifft eine Kapselhalterung für übliche Arzneimittelkapseln, insbesondere für die Verwendung in Verbindung mit Inhalationsgeräten, in denen mit mikronisiertem Arzneimittel gefüllte Kapseln benutzt werden.

Inhalationsgeräte für die Pulverinhalation, mit deren Hilfe Arzneimittelpulver aus Kapseln inhaliert werden können, sind weithin in Gebrauch. Die älteren Versionen waren so ausgelegt, daß sie nur eine Kapsel aufnehmen konnten (DE-A 14 91 715; DE-A 33 45 722).

Um den, Patienten das Nachfüllen zu erleichtern wurden später Inhalatoren entwickelt in denen mehrere Kapseln bereitgehalten werden können (z.B. DE-A 39 27 170). Die zur Aufnahme der Kapseln bestimmten Kammern oder Vertiefungen haben einen größeren Durchmesser als die Kapseln.

Beim Hantieren mit dein geöffneten Inhalator können die Kapseln leicht herausfallen, weil sie in den einzelnen Kammern nicht fixiert sind. Besonders beim Asthmaanfall, bei dem, der Patient rasch die Inhalation vornehmen will, ist es störend, wenn die Kapseln nur lose im Inhalator liegen, so daß der Patient auch darauf achten muß, daß die Kapseln während der Benutzung nicht aus dem Gerät fallen. Andererseits ist es erwünscht, entleerte Kapseln sofort nach Gebrauch zu entfernen, ohne daß gleichzeitig die noch gefüllten Kapseln herausfallen, damit jeweils ohne weiteres festzustellen ist, wie groß der Vorrat an gefüllten Kapseln im Gerät noch ist.

Die Erfindung schlägt nun eine Kapselhalterung vor, die einerseits die Kapseln so fixiert, daß sie beim üblichen Hantieren damit nicht herausfallen, andererseits aber eine leichte Entnahme ermöglicht, und zwar unabhängig davon, mit welchem Ende die Kapseln in die Vertiefungen gesteckt werden.

Bekanntlich bestehen die zu therapeutischen Zwecken verwendeten Arzneimittelkapseln aus zwei Teilen; jeder der Teile hat zylindrische Form und hat an einem Ende einen halbkugelförmigen Abschluß. Der Innendurchmesser der beiden Zylinder ist so gewählt, daß beim Zusammenstecken mit den offenen Seiten eine relativ feste Verbindung erreicht wird. Der Außendurchmesser der beiden zylindrischen Teile ist verschieden. Bei einer handelsüblichen Kapsel der Größe 3 beispielsweise beträgt der Durchmesser des Oberteils 5,83, der Durchmesser des Unterteils 5,57 mm. Die Kapseln bestehen aus elastischem Material, vorzugsweise aus Hartgelatine.

Zylindrische Vertiefungen, die den äußeren Abmessungen der Kapseln entsprechen, lösen die erfindungsgemäße Aufgabe nicht, weil sie jeweils für eine Kapselhälfte zu eng oder zu weit sind.

Es wurde nun eine Kapselhalterung für Arzneimittelkapseln entwickelt, in welche die Kapseln sowohl mit ihrem Ober- als auch mit ihrem Unterteil gleich tief eingesteckt werden können, wobei sie einerseits ausreichend fest sitzen, um nicht herauszufallen, andererseits leicht genug entnehmbar sind, damit die Teile der Kapseln bei der Entnahme nicht versehentlich auseinandergezogen werden.

Die erfindungsgemäße Halterung besteht aus einer Vertiefung, in welche die Kapsel in Richtung ihrer Längsachse hineingesteckt wird, wobei die Wandung der Vertiefung mindestens drei parallel zur Längsachse in ungleichem Abstand voneinander angeordnete Rippen aufweist, zwischen denen die Kapsel unter Verformung eingeklemmt wird.

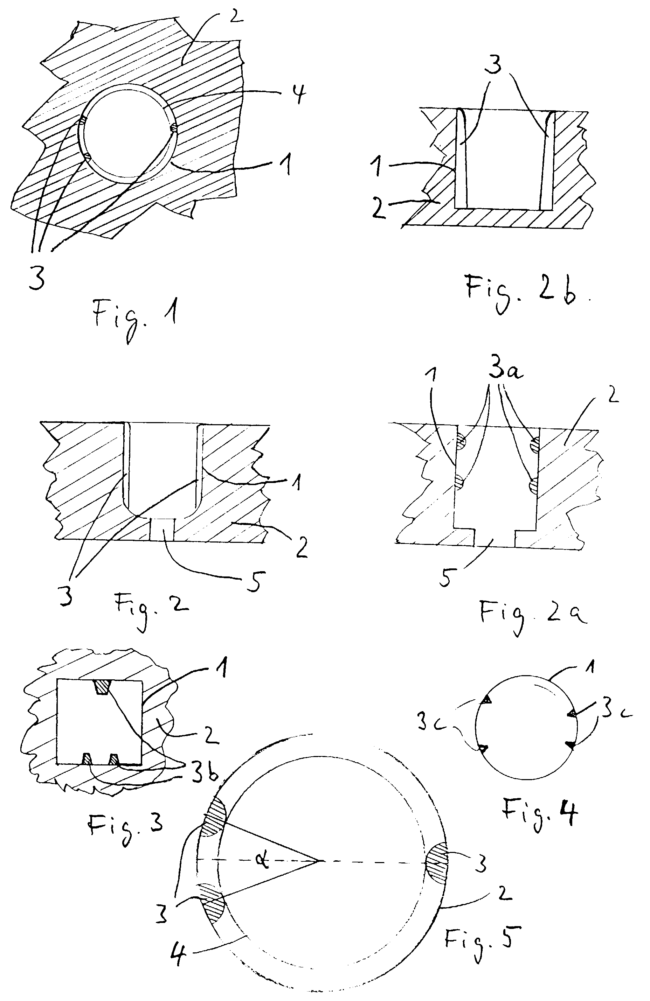

Eine Kapselhalterung dieser Art ist in den Figuren 1 bis 6 dargestellt.
Figur 1 zeigt eine erfindungsgemäße Kapselhalterung im Querschnitt.
Die Vertiefung 1 mit kreisförmigem Querschnitt in der Platte 2 weist drei Rippen 3 auf, von denen zwei verhältnismäßig nahe beeinander liegen und die dritte gegenüber. Zwischen den Rippen läßt sich ein Kreis 4 einbeschreiben, dessen Durchmesser geringfügig kleiner ist als der Außendurchmesser des unteren (dünneren) Kapselteils. Beim Einschieben zwischen die Rippen wird die Kapsel leicht verformt. Zweckmäßigerweise werden die Rippen an ihrem äußeren Ende abgerundet oder abgeschrägt, was das Einführen der Kapseln erleichtert und eine Beschädigung der Kapseln beim Einführen verhindert.

Der Querschnitt der Rippen läßt viele Abwandlungen zu, meist ist er halbkreisförmig bzw. abgerundet, er kann aber auch z.B. dreieckig oder viereckig sein, wobei die Höhe der Rippen vorzugsweise gleich ist, aber nicht gleich sein muß. Entsprechendes gilt für die Breite der Rippen. Die Fläche, mit der sich Kapseln und Rippen berühren, sollte relativ klein sein, damit auch nach längerem Aufbewahren in der Halterung die Kapsel noch gut entnommen werden kann und nicht verklebt. Die Vertiefung kann sich (bei konstanter Rippenhöhe) etwas verengen, das heißt leicht konisch geformt sein; auch können die Rippen zum unteren Ende der Vertiefung hin etwas höher werden, so daß auch in diesem Fall (bei konstantem Querschnitt der Vertiefung) eine Verringerung des Zwischenraums zwischen den Rippen erreicht wird.

Figur 2 zeigt einen senkrechten Schnitt entlang der Längsachse einer erfindungsgemäßen Kapselhalterung, wobei in der Vertiefung 1 der Platte 2 die Rippen 3 zu erkennen sind. Die Vertiefung 1 besitzt im allgemeinen unten eine Öffnung 5, welche die Reinigung erleichtert.

Wie Figur 2a zeigt, können die Rippen auch durch entsprechend angeordnete Noppen 3a ersetzt werden.

In Figur 2b wächst die Höhe des Rippenquerschnitts nach unten, so daß sich nach unten hin ein geringerer Abstand der Rippen voneinander ergibt.

Figur 3 zeigt ein Beispiel für eine Kapselhalterung mit rechteckigem Querschnitt und einer andersartigen Gestaltung der Rippen (3b).

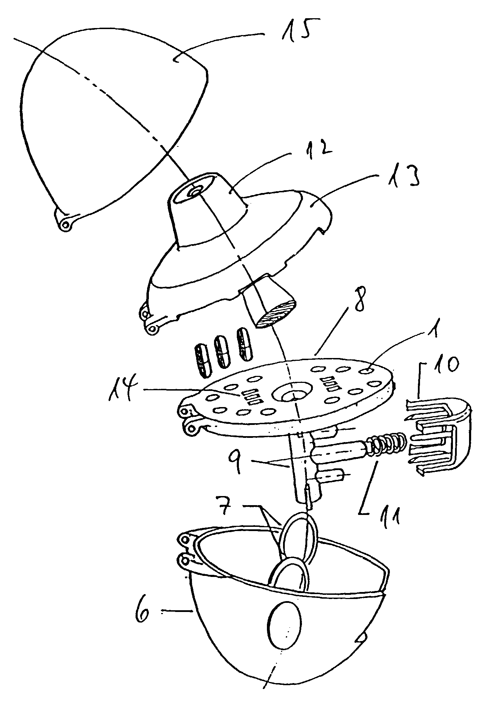

Eine größere Zahl von Rippen läßt Figur 4 erkennen, wobei die Rippen 3c einen dreieckigen Querschnitt haben.

Um (unter sonst gleichen Voraussetzungen) eine ausreichende Verformung zu ermöglichen, sind die Rippen - bezogen auf den einbeschriebenen Kreis - nicht in gleichen Abständen angeordnet. Dies soll in Figur 5 veranschaulicht werden. Der Winkel α zwischen den beiden enger benachbarten Rippen 3 beträgt etwa 30 bis 60°, vorzugsweise 35 bis 50°. Besonders bewährt sich ein Winkel von ca. 40°. Die gegenüberliegende Rippe liegt auf dem Durchmesser, der den Winkel α halbiert oder ist allenfalls geringfügig seitlich versetzt. Bei einem Winkel von z. B. 40° zentriert sich die Kapsel beim Einführen von selbst, während sie bei zu kleinem Winkel schräg stehen kann, so daß u. U. das herausnehmen behindert sein kann, insbesondere wenn mehrere Halterungen dicht nebeneinander vorgesehen sind.

Aus Figur 6 ist zu entnehmen, wie ein Inhalator aufgebaut sein kann, in den erfindungsgemäße Kapselhalter integriert sind.

In einem Unterteil 6 mit zwei Fenstern 7 befindet sich die Platte 8, die mit der Inhalationskammer 9 verbunden ist und in der sich auch 12 Kapselhalterungen 1 befinden. Zum Öffnen der Kapseln in der Kapselkammer 9 dient der mit zwei speziell geschliffenen Nadeln versehene Knopf 10, der gegen den Druck der Feder 11 eingedrückt wird und dabei die Kapsel in der Kammer an zwei Stellen aufschneidet bzw. aufsticht. Beim Inhalieren durch das Gerät mittels des Mundrohrs 12, das mit dem Oberteil 13 verbunden ist, gelangt die Luft durch die Öffnungen 14 in das Unterteil 6 und von dort am unteren Ende in die Kapselkammer 9, die so gebaut ist wie die Kapselkammer in dem Inhalator gemäß DE-A 3927170. Das Gerät wird durch einen Deckel verschlossen, der klappbar mit dem Unterteil 6, der Platte 8 und dem Oberteil 13 verbunden ist, so daß bei geschlossenem Deckel Staub nicht in das Gerät eindringen kann.

## Patentansprüche

1. Inhalator für die Inhalation pulverförmiger Arzneimittel aus Kapseln gekennzeichnet durch
ein Unterteil 6, enthaltend zwei Fenster 7 und eine Platte 8, in der sich 12 Kapselhalterungen 1 sowie Lufteinlaßöffnungen 14 befinden,
eine mit der Platte 8 verbundene Inhalationskammer 9, an der ein mit zwei geschliffenen Nadeln versehener, gegen eine Feder 11 beweglicher Kopf 10 vorgesehen ist,
ein mit einem Oberteil 13 verbundenes Mundrohr 12,
sowie einen Deckel 15, der klappbar mit dem Unterteil 6, der Platte 8 und dem Oberteil 13 verbunden ist.
